# NEUE EUROPÄISCHE PATENTSCHRIFT

(11) **EP 2 532 370 B2**
(45) Veröffentlichungstag und Bekanntmachung des Hinweises auf die Entscheidung über den Einspruch: **04.09.2024**
(45) Hinweis auf die Patenterteilung: 22.06.2016
(21) Anmeldenummer: 12183372.7
(22) Anmeldetag: 18.12.2009
(51) Int. Cl.: A61K 49/00, A61M 5/178

(54) **Farbstofflösung**
Dye solution
Solution comprenant un colorant

(30) Priorität: 19.12.2008 DE 102008064065
(43) Veröffentlichungstag der Anmeldung: 12.12.2012
(62) Teilanmeldung aus: 09801410.3
(73) Patentinhaber: FLUORON GMBH, 89077 Ulm (DE)
(72) Erfinder: Lingenfelder, Christian, 89081 Ulm (DE); Theisinger, Bastian, 68239 Mannheim (DE); Hiebl, Wilfried, 89257 Illertissen (DE); Hagedorn, Nadine, 89134 Blaustein (DE)
(74) Vertreter: Schiweck Weinzierl Koch Patentanwälte Partnerschaft mbB

(56) Entgegenhaltungen:
- EP-A1- 1 132 065
- WO-A1-2004/035091
- WO-A1-2004/035091
- WO-A1-2010/060018
- WO-A2-03/057259
- US-A1- 2003 097 117
- ÜNLÜ K ET AL.: "Gentian violet solution for staining the anterior capsule", J CATARACT REFRACT SURG, vol. 26, 1 August 2000 (2000-08-01), pages 1228 - 11232, XP002605866
- ALCON LABORATORIES: "BSS Plus", Retrieved from the Internet <URL:http://ecatalog.alcon.com/pi/BSSplus250_us_en.pdf> [retrieved on 20101008]
- DE PAULA FIOD COSTA, E. ET AL.: "Vital dyes and light sources for chromvitrectomy: comparative assessment of osmolarity, PH and spectrofotometry", - 8 August 2008 (2008-08-08), Retrieved from the Internet <URL:www.iovs.org/content/early/2008/08/08/iovs.08-2285.pdf> [retrieved on 20130402]
- LECHNER, T AND MÜLLER B.K: "Determination of the density of various dye solutions at 25°C and 35°C", 5 April 2013 (2013-04-05), pages 1 - 3
- LESNICK-OBERSTEIN, S.Y ET AL.: "IMPROVING THE STAINING CHARACTERISTICS OF TRYPAN BLUE IN VITREo-retinal surgery", INVESTIGATIVE OPHTHALMOLOGY AND VISUAL CCIENCE, vol. 45, no. E-ABSTRACT 1984, - 1 January 2004 (2004-01-01)
- PETER STALMANS ET AL: "Toxic effect of indocyanine green on retinal pigment epithelium related to osmotic effects of the solvent", AMERICAN JOURNAL OF OPHTHALMOLOGY, vol. 134, no. 2, 1 August 2002 (2002-08-01), pages 282 - 285, XP055187000, ISSN: 0002-9394, DOI: 10.1016/S0002-9394(02)01468-X
- DR. M.K. SCHMID: "A new Method to Improve the Application of Dyes on the Retinal Surface Without Fluid-Air Exchange", VIDEO PRESENTATION EVRS CONGRESS IN PRAGUE, 9 September 2008 (2008-09-09)
- Transcript of the presentation by Martin K Schmid

## Beschreibung

### Gebiet der Erfindung

Die Erfindung betrifft eine wasserbasierte biocompatible Zubereitung zum in vivo selektiven Anfärben von Membrana limitans interna (ILM) und/oder von epiretinalen Membranen (ERM) im menschlichen oder tierischen Auge gemäß Anspruch 7.

Ferner betrifft die Erfindung eine wasserbasierte, biokompatible Zubereitung zur Verwendung bei der chirurgischen Behandlung am Auge, umfassend das selektive Anfärben der ILM und/oder ERM im menschlichen oder tierischen Auge und Entfernen der gefärbten Membran gemäß Anspruch 1.

### Hintergrund der Erfindung

Erkrankungen des Auges, wie Grauer Star (Katarakt), Glaukom (Gruner Star), altersbedingte Makuladegeneration und Diabetes-bedingte Retinopathie, sowie Netzhautveränderungen bzw. Netzhautablösungen nehmen zu, bedingt unter anderem durch die höhere Lebenserwartung. Zu der Behandlung dieser und weiterer Augenkrankheiten ist häufig eine Vitrektomie indiziert. Dabei muss sichergestellt werden, dass die Netzhaut möglichst wenig geschädigt wird. Eine Vorsichtsmaßnahme besteht darin, bei der Vitrektomie die Membrana limitans interna (ILM) und ggf. epiretinale Membranen von der Netzhaut zu entfernen, um die Makula von den vermuteten intravitrealen Zugkräften zu entlasten. Dazu werden die Membranen mit einer Pinzette von der Netzhaut abgezogen. Für den Chirurgen ist es notwendig, dass er möglichst genau zwischen Netzhaut und abzuziehender Membran unterscheiden kann. Dazu sollen die abzuziehenden Membranen über eine möglichst spezifische Anfärbung sichtbar gemacht werden. Für eine Anfärbung geeignete Farbstoffe müssen viele Kriterien erfüllen. Sie müssen biokompatibel und untoxisch sein und dürfen die Zellen nicht schädigen, sie sollten wasserlöslich sein, sie sollten möglichst spezifisch anfärben und leicht wieder ausspülbar sein. Es wurden bereits Farbstoffe und Verfahren zur Anfärbung der genannten Membranen beschrieben, die aber noch nicht voll befriedigen.

So beschreibt US 7,014,991 ein Verfahren zur Anfärbung von okularen Strukturen im menschlichen Auge, wobei das Anfärben durch Einspritzen des Farbstoffes Indigotindisulfat in das entsprechende Gewebe erfolgt. Indigotindisulfat ist jedoch cytotoxisch.

Weitere Farbstoffe, wie Brillantblau G, Brilliant Blau R, Patentblau V, oder Methylenblau wurden ebenfalls zur Verwendung im Auge vorgeschlagen.

WO 03/057259 beschreibt Lösungen, die Indocyaningrün beinhalten und zur Darstellung von Gefäßen verwendet werden.

US 2003/0097117 beschreibt Vorrichtungen zum Einbringen von Farbstofflösungen in das Auge.

EP 1132065 beschreibt gefärbte viskoelastische Zusammensetzungen, die bei Augenoperationen als Hilfsmittel, z.B. zum Schutz intra-okularen Gewebes, als Platzhalter oder zur Erleichterung intraokularer Manöver, beispielsweise bei einer kontrollierten Kapsulorhexis eingesetzt werden können.

Unlu et al. (2000), J. Cataract Refract. Surg. 26(8), 1228-1232 beschreibt die Verwendung des Farbstoffs Gentianaviolett zum Färben der vorderen Linsenkapsel bei der Kataraktoperation.

WO 2004/035091 beschreibt Farbstoffe, insbesondere Triphenylmethanfarbstoffe zum Anfärben von Geweben, insbesondere Membranen im Auge, z.B. die ILM.

WO 2010/060018 beschreibt Farbstoffe für die Phototherapie.

Costa et al. (2009), Invest. Ophthalmol. Vis. Sci. 50(1), 385-391 beschreibt Lösungen von Wasser, BSS oder 5% Glukose, in denen Farbstoffe gelöst sind, wobei die Lösungen auf in vitro Aspekte wie z.B. pH, Osmolarität oder Photostabilität untersucht werden.

WO 2006/062233 beschreibt Brilliant Blau G Farbstoffe zum Anfärben der ILM und des Linsensacks als Hilfsmittel bei Augenoperationen.

Lesnik-Oberstein et al. (2004), ARVO, Invest. Ophthalmol. Vis. Sci., 45, E-Abstract 1984 beschreibt eine mit 2,5% bzw. 5% Glukose versetzte Lösung mit Trypan blau, die zum Färben der ERM bei chirurgischen Eingriffen verwendet werden kann. Ebenso beschreibt Lesnik-Oberstein et al. (2007), Br. J. Ophthalmol. 91, 955-957 eine mit Glukose versetzte Trypanblaulösung zum Färben der ERM, wodurch auf die Durchführung eines sogenannten Air-Fluid-Exchange (AFX) vor dem Färben verzichtet werden kann.

WO 99/58159 beschreibt verschiedene Farbstoffe, z.B. Gentianaviolett oder Trypanblau als Hilfsmittel bei Augenoperationen.

Rodrigues et al. (2005), Ophthalmologica 219, 251-262 beschreibt Indocyaningrun-Lösungen zur Verwendung als Färbemittel bei der Behandlung von Makulalöchern und schlagt vor, diese Lösungen mit einem Viskoelastikum oder Perfluorierten Carbonsäuren zu versetzen, um den Austritt des Farbstoffs zu verhindern, so dass er in der Lösung verbleibt.

Stalmans et al. (2002), American J. Ophthalmol., 134(2), 282-285 beschreibt Lösungen, die den Farbstoff Trypanblau und 5% Glukose enthalten und zum Färben der ILM verwendet werden können.

Während der Vitrektomie oder des chirurgischen Eingriffs wird die Augenhöhle mit einer Spullösung gespült. Ein Problem der bisher bekannten Farbstofflösungen besteht nun darin, dass durch die Spullösung die Farbstofflösung verteilt, verdünnt und ausgespült wird. Dies hat mehrere Nachteile. Zum einen wird die Sicht des Chirurgen getrübt, wenn die Spullösung gefärbt ist. Zum anderen ist mehr Farbstofflösung erforderlich, als nur zum Anfärben der Membrannotwendig wäre.

Um diesen Nachteil zu überwinden, wurde schon vorgeschlagen, der Farbstofflösung ein Verdickungsmittel, wie z.B. Hyaluronsäure, zuzusetzen, das die Viskosität der Farbstofflösung erhöht. Die Erhöhung der Viskosität soll dazu führen, dass der Farbstoff aufgrund verminderter Beweglichkeit, also durch sterische Hinderung, nicht so stark in die Spullösung übertritt und eher in die Nähe der anzufärbenden Membran gelangt. Allerdings führt die hohe Viskosität der Farbstofflösung dazu, dass der Farbstoff nunmehr aus dieser heraus schlecht auf die Membran übertreten kann, so dass wiederum der Bedarf an Farbstofflösung höher ist als die Menge, die nur zur Anfärbung der Membran notwendig wäre.

Es war daher Aufgabe der Erfindung, eine Zubereitung bereitzustellen, die Membranen speziell anfärben kann, insbesondere die zu entfernenden Membranen wie Membrana limitans interna (ILM) und/oder epiretinale Membranen (ERM), im menschlichen oder tierischen Auge selektiv anfärben kann, die sich gut applizieren lasst, direkt nach der Applikation zur Membran wandert und sich dort verteilt, ohne die Spullösung zu stark anzufärben. Weiterhin soll eine Zubereitung bereitgestellt werden, die weder zu lokalen Reizungen noch Schädigungen der Netzhaut führt, nicht cytotoxisch ist, sondern gut vertragen wird.

Diese Aufgabe wird gelöst mit einer Zubereitung bzw. einer Zubereitung zur Verwendung bei der chirurgischen Behandlung am Auge, wie sie in den Ansprüchen, insbesondere in Anspruch 1 definiert ist.

Die Unteransprüche enthalten vorteilhafte Weiterbildungen.

### Detaillierte Beschreibung der Erfindung

Überraschenderweise wurde gefunden, dass eine Zubereitung, die mindestens einen Farbstoff enthält, der ausgewählt ist aus Triphenylmethanfarbstoffen dann ein effektives und selektives Anfärben der ILM und/oder der ERM zulässt, wenn die Dichte der Zubereitung in einem Bereich von 1,01 g/cm³ bis 1,5 g/cm³, bevorzugt 1,01 g/cm³ bis 1,3 g/cm³ eingestellt wird, wobei zum Einstellen der Dichte kein Monosaccharid oder reduzierendes Disaccharid verwendet wird, wobei das Mittel ein nicht-reduzierendes Disaccharid, ein Polysaccharid oder ein neutrales Polymer oder eine Kombination davon ist, und wobei die Osmolarität der hergestellten Lösungen im Bereich von 280-330 mosmol/L liegt..

Es wurde gefunden, dass eine Farbstofflösung mit übergegenüber dem Wasser erhöhter Dichte dann, wenn sie im Rahmen einer chirurgischen Behandlung am Auge in den Bereich der Augenhöhle eingespritzt wird, absinkt, wodurch ein schnelles Vermischen mit der Spullösung vermieden wird, und sich nach dem Absinken auf der Membran verteilt und diese anfärbt. Dadurch wird vermieden, dass der Farbstoff mit der Spullösung zu schnell weggespült wird und auch, dass der Farbstoff das Sichtfeld eintrübt.

Die erfindungsgemäße Zubereitung basiert auf Wasser als Lösungsmittel, wobei weitere Lösungsmittel gegebenenfalls in geringeren Anteilen enthalten sein können, sofern sie mit Wasser homogen vermischbar sind und biologisch kompatibel sind. In Betracht kommen hier ein- und mehrwertige Alkohole, wie sie im medizinischen Bereich auch Anwendung finden. Falls ein weiteres Lösungsmittel verwendet wird, ist dieses besonders bevorzugt ein Glycol oder Glycerin. Auch Mischungen der genannten Lösungsmittel kommen in Betracht. Falls dem Wasser ein Lösungsmittel zugemischt wird, so sollte dieses in einem Anteil von nicht mehr als 20 Gew.-%, bevorzugter nicht mehr als 10 Gew.-% verwendet werden. Bevorzugt ist die Zubereitung einer istotonischen Lösung.

Außer Wasser als Lösungsmittel und dem Farbstoff, der unten naher ausgeführt wird, enthält die erfindungsgemäße Zubereitung als wesentlichen Bestandteil ein die Dichte einstellendes Mittel. Das die Dichte einstellende Mittel muss biologisch kompatibel sein, darf nicht toxisch sein und muss mit Wasser homogen vermischbar sein, ggf. nach Zugabe einer geringen Menge eines Löslichkeitsvermittlers wie Alkohol, so dass eine klare transparente Lösung entsteht. Außerdem muss sie mit dem Farbstoff kompatibel sein, d.h. darf nicht die Löslichkeit des Farbstoffs in erheblichem Ausmaß beeinträchtigen. Bei der Einstellung der Zubereitung ist auch die Osmolarität zu beachten, um keine durch Osmose bedingten Schäden am Gewebe hervorzurufen. Die Osmolarität liegt in einem Bereich von 280-330 mosmol/L, bevorzugt bei 300 mosmol/L.

In Betracht kommen daher mit Wasser kompatible Flüssigkeiten, deren Dichte über der Dichte des Wassers liegt.

Ein Mittel, mit dem die Dichte eingestellt werden kann, ist ein Polysaccharid. Polysaccharide eignen sich zur Erhöhung der Dichte und sind gut verfügbar. Außerdem sind sie toxikologisch unbedenklich und biologisch kompatibel. Unter Polysacchariden werden hier Moleküle verstanden, die aus mehr als zwei, bevorzugt mehr als 5, besonders bevorzugt mehr als 10 Saccharideinheiten aufgebaut sind. Obwohl allgemein Mono- und Disaccharide die Dichte erhöhen können, werden erfindungsgemäß nur nicht-reduzierende Disaccharide zur Erhöhung der Dichte eingesetzt. Die Verwendung von Monosacchariden und reduzierenden Disacchariden kann zu unerwünschten Wirkungen führen, so können diese z.B. in der zur Erhöhung der Dichte notwendigen Menge cytotoxisch sein. Erfindungsgemäß geeignete nicht-reduzierende Disaccharide sind Saccharose oder Trehalose. Als geeignete Polysaccharide können lösliche Stärkederivate, wie Hydroxyethylstärke und Dextran genannt werden. Als Polysaccharide sind solche Verbindungen geeignet, die neutral sind, nicht reduzierend wirken und sich in wässriger Lösung nicht abbauen.

Weitere Mittel zur Einstellung der Dichte sind neutrale Polymere wie Polyether, Polyvinylalkohol, Polyester, Polyacrylsäure Copolymer, Polyvinylpyrrolidon.

Auch Kombinationen der genannten Mittel sind gut geeignet, um die Dichte der erfindungsgemäßen Zubereitung einzustellen.

Die Dichte der Zubereitung kann dabei nach jeder gängigen Methode bestimmt werden, wie sie dem Fachmann allgemein bekannt ist.

Es hat sich herausgestellt, dass die Erhöhung der Dichte auf 1,01 g/cm³ bereits den gewünschten Effekt bewirkt, nämlich dass die Farbstofflösung nach Applikation in die Augenhöhle schnell nach unten sinkt und sich dort dann auf der Membran verteilen kann. Dadurch gelingt ein selektives Anfärben der Membran, ohne die Sicht des Operateurs zu verschlechtern. Ein Dichteunterschied von weniger als 0,01 g/cm³ bezogen auf Wasser ist nicht mehr ausreichend, um die Farbstoffzubereitung gezielt absinken zu lassen. Das Absinken erfolgt dann so langsam wie bei den Zubereitungen des Standes der Technik und führt zu den oben aufgeführten Problemen. Wenn die Dichte der Zubereitung größer als 1,5 g/cm³ ist, so kann es aufgrund der Dichte zu einer Schädigung der sehr empfindlichen Netzhaut kommen.

Ein weiterer wichtiger Bestandteil der erfindungsgemäßen Zubereitung ist der Farbstoff, der ein Triphenylmethanfarbstoff ist. Als Farbstoff können solche Verbindungen eingesetzt werden, die die ILM und/oder ERM spezifisch und gezielt anfärben können, so dass sich die Membran optisch von der Netzhaut unterscheidet. Weiterhin muss der Farbstoff in dem Wasser bzw. der Mischung aus Wasser und einem weiteren Lösungsmittel löslich sein. Er darf weder toxisch, insbesondere cytotoxisch bzw. zellschädigend sein, noch Schaden an der Netzhaut hervorrufen oder durch Lichtreaktionen toxische Wirkungen entwickeln wie ICG oder Trypanblau. Außerdem sollte er ein gutes Färbevermögen haben, um die Menge des Farbstoffs gering halten zu können.

Als vorteilhaft haben sich Farbstoffe aus der Gruppe der Triphenylmethanfarbstoffe, wie Brillantblau G, Brilliant Blau R, Brilliant Blau FCF, Patentblau V, Bromphenolblau, Lissamine Grün SF, Lissamine Grün G, Fast Green, Methylgrün, Brilliantsäuregrün, Commassie Violet R 200, Rosanillin.

Bevorzugt werden Brillantblau G, Brilliantblau R, Brilliantblau FCF, Patentblau V, Methylgrün, Commassie Violet R 200, Bromphenolblau und/oder Chicagoblau verwendet. Von den Triphenylmethanfarbstoffen sind Brillantblau G, Coomassie Violet R 200 und Chicagoblau besonders bevorzugt. Von den Brillantblau-Farbstoffen ist Brilliantblau G aufgrund seines besonders guten Färbevermögens bevorzugt. Er kann in einer Konzentration von weniger als 0,3 g/l eingesetzt werden. Diese geringe Konzentration führt bereits zu einer ausreichenden selektiven Färbung von ILM und/oder ERM.
Vorzugsweise enthält die erfindungsgemäße Zubereitung zusätzlich einen Farbstoff ausgewählt aus der Gruppe bestehend aus Azofarbstoffen, Cyaninfarbstoffen und/oder Naturfarbstoffen oder deren Mischungen .

Um die vorteilhaften Eigenschaften der erfindungsgemäßen Zubereitung weiter zu verbessern, kann der Zubereitung zusätzlich noch ein die Viskosität einstellendes Mittel zugefügt werden. Es hat sich gezeigt, dass der Zusatz eines die Viskosität der erfindungsgemäßen Zubereitung erhöhenden Mittels eine Verbesserung der Kohäsivitat bewirken kann, sodass die mit der erfindungsgemäßen Zubereitung erhaltenen Vorteile noch verstärkt werden. Die applizierte Zubereitung, die aufgrund der höheren Dichte schneller absinkt, wird noch weniger in der Spullösung verteilt, da sie aufgrund der erhöhten Viskosität bis zum Auftreffen auf die Membran zusammengehalten wird. Da ein vorteilhafter Effekt aber bereits durch Einstellung der Dichte erreicht wird, muss die Viskosität nicht so stark erhöht werden, dass dies zu Problemen führt, wie sie im Stand der Technik bestehen. Bereits eine geringe Anhebung der Viskosität führt dazu, dass die aus dem Applikationsgerat austretenden Tropfen eine stabilere Einheit bilden und damit weniger leicht verdünnbar sind, was verhindert, dass der in der Zubereitung eingebettete Farbstoff ausgeschwemmt wird. Dadurch wird der Farbstoff erst am Applikationsort durch Kapillareffekte auf der Membran freigesetzt, die sich dadurch einfärbt. Auf diese Weise kann der Farbstoff gezielt zur Membran gebracht werden.

Als viskositätsregulierende biokompatible Mittel, also Mittel, die die Viskosität einstellen, können aus der folgenden Gruppe ein oder mehrere eingesetzt werden: Polyether, Polyvinylalkohol, Polyester, Polyacrylsäure Copolymer, Polyvinylpyrrolidon, und andere Polymere, mehrwertige Alkohole wie Glycerin, Propylenglycol, Butylenglycol, wasserlösliche Cellulosederivate wie Methylcellulose, Xanthan Gummi, Stärke, Hyaluronsäure und deren jeweilige Derivate, Chondroitinsulfat und Natriumsulfat. Als die Viskosität regulierende Mittel können auch solche verwendet werden, die nicht nur die Viskosität, sondern gleichzeitig auch die Dichte erhöhen. In diesem Fall ist es wichtig, darauf zu achten, dass beide Parameter, d.h. sowohl die Viskosität als auch die Dichte, im gewünschten Bereich liegen. Mit anderen Worten darf ein die Dichte beeinflussendes und die Viskosität regulierendes Mittel nicht in einer solchen Menge eingesetzt werden, dass die fertige Zubereitung dann eine Dichte von mehr als 1,5 g/cm³ aufweist. Die geeigneten Mengen können jedoch vom Fachmann leicht, gegebenenfalls mit Routineversuchen, festgestellt und die entsprechenden Werte in der Zubereitung eingestellt werden.

Besonders geeignet als viskositätsregulierende Mittel sind solche, die eine gewisse Affinität zu dem erfindungsgemäß verwendeten Farbstoff aufweisen und sich durch ein hohes Spreitvermögen auszeichnen. Überraschenderweise wurde gefunden, dass Butylenglycol ein Mittel ist, mit dem die Viskosität reguliert werden kann und das zu einem guten Spreitvermogen führt. Ein Zusatz von Butylenglycol kann daher dafür sorgen, dass die applizierte Zubereitung nach unten sinkt und, sobald sie die Membran erreicht hat, sich dort ausbreitet und die Membran schnell anfärbt. Dies wird, ohne an eine Theorie gebunden zu sein, damit erklärt, dass einerseits Butylenglycol eine Affinität zu Membranen hat und andererseits aufgrund lipophiler Gruppen den Farbstoff gut adsorbiert. Wenn die butylenglycol- und farbstoffhaltige Zubereitung die Membran erreicht, sorgt das Butylenglycol dafür, dass sich der Farbstoff schnell auf der Membran verteilen kann

Die Viskosität der erfindungsgemäßen Zubereitung wird bevorzugt so eingestellt, dass die Scherviskosität bei 25 °C und einer Scherrate von 10 s⁻¹ in einem Bereich vom 1 bis 500 mPas liegt. Bevorzugt wird die Scherviskosität bei 25 °C und einer Scherrate von 10 s⁻¹ auf einen Bereich von 50 bis 275 mPas eingestellt. Das Einstellen der Viskosität kann mit den bereits genannten viskositätsregulierenden Mitteln erzielt werden. Liegt die Viskosität, unter den angegebenen Messbedingungen in einem Bereich von 1 bis 500 mPas, so werden die mit der erfindungsgemäßen Zubereitung erzielten Effekte deutlich verstärkt. Die den selektiv färbenden Farbstoff enthaltende Zubereitung sinkt schnell ab, ohne dass der Farbstoff in nennenswertem Umfang mit der Spullösung ausgewaschen wird. Der Farbstoff wird somit erst am Applikationsort durch Kapillareffekte auf der Membran freigesetzt, die sich dadurch einfärbt. Ist die Viskosität unter den angegebenen Messbedingungen geringer als 1 mPas, so kann der Effekt des schnellen Absetzens der erfindungsgemäßen Zubereitung nicht zusätzlich verstärkt werden. Es besteht die Möglichkeit, dass wenigstens ein Teil des Farbstoffes vor dem Anfärben der Membran mit der Spullösung ausgetragen wird und somit nicht mehr für das Anfärben der Membran zur Verfügung steht. Liegt die dynamische Viskosität bei 25 °C und einer Scherrate von 10 s⁻¹ dagegen oberhalb von 500 mPas, so ist die Viskosität der Zubereitung so hoch, dass der Farbstoff nicht optimal aus den sich bildenden Tröpfchen freigesetzt werden kann. Die Fähigkeit der Farbstoffzubereitung zu spreiten, was eine schnelle, homogene Anfärbung der Membran bewirkt, wird dadurch deutlich reduziert. Die Membran wird nicht optimal mit der Farbstoffzubereitung benetzt und damit auch nicht so deutlich gefärbt. Ein besonders gutes Anfärbeergebnis wird erreicht, wenn die dynamische Viskosität bei 25 °C und einer Scherrate von 10 s⁻¹ in einem Bereich von 50 bis 275 mPas liegt.

Es wurde gefunden, dass bei Verabreichung von Farbstofflösungen in das Auge Probleme auftreten können. Wenn die Farbstofflösung mit den üblicherweise verwendeten Spritzen verabreicht wird, wird der Druck, der beim Einspritzen erzielt wird, zu hoch, so dass der Farbstoff hinter die Netzhaut geraten kann.

Vorzugsweise werden Spritzen verwendet, bei denen Kanülendurchmesser, das Verhältnis von Zylinderdurchmesser zu Kanülendurchmesser und das Aspektverhältnis so angepasst sind, dass Schäden vermieden werden. Erfindungsgemäß werden bevorzugt Spritzen verwendet, bei denen der Kanülendurchmesser sehr klein ist, um die Schädigung im Auge möglichst gering zu halten. Weiterhin wird der Zylinderdurchmesser so an den Kanülendurchmesser angepasst, dass das Auftreten eines Venturi-Effekts weitgehend vermieden wird. Mit anderen Worten muss bei der zur Verabreichung vorgesehenen Spritze auch der Durchmesser des Zylinders möglichst klein sein, sodass das Verhältnis von Zylinderdurchmesser zu Kanülendurchmesser im Bereich von 10 bis 2 zu 1 bis 0,2, bevorzugt 20 zu1 bis 4 zu 1, besonders bevorzugt 16 zu 1 bis 8 zu 1 liegt. Außerdem sollten die Spritzenzylinder ein Aspektverhältnis, d.h. Verhältnis von Zylinderlänge zu Zylinderdurchmesser, in einem Bereich von 15 bis 5 zu 1 haben.

Weiterhin beschrieben ist ein Kit, der eine Spritze mit Zylinder und Kanüle enthaltend eine Farbstoffzubereitung zum selektiven Anfärben der Membrana limitans interna und/oder von epiretinalen Membranen im menschlichen oder tierischen Auge, umfasst, wobei das Verhältnis von Zylinderdurchmesser zu Kanülendurchmesser im Bereich von 10 - 2 zu 1 - 0,2, bevorzugt 20:1 bis 4:1, besonders bevorzugt 16:1 bis 8:1 liegt. Bevorzugt ist das Verhältnis von Zylinderlänge zu Zylinderdurchmesser in einem Bereich von 15 bis 5 zu 1. Als wesentlichen Bestandteil weist der Kit somit eine Spritze auf, deren Zylinderdurchmesser an den Durchmesser der Kanüle angepasst ist. Es wurde gefunden, dass bei einem kleineren Verhältnis der Durchmesser sich im Innenraum vor der Kanüle kein Druck aufbauen kann, so dass ein gleichmäßiges Applizieren, also ein Applizieren mit gleichmäßigem Druck und gleichbleibender Geschwindigkeit der erfindungsgemäßen Zubereitung gewährleistet ist. Bevorzugt enthält der Kit eine erfindungsgemäße Farbstoffzubereitung wie oben beschrieben.

Für den Kit, bzw. dessen Spritze wird bevorzugt eine Kanüle mit 19 bis 27 gauge, besonders bevorzugt 23 oder 25 gauge verwendet. Kanülen mit 19 bis 27 gauge sind für Injektionen in das Auge geeignet. Ihre Austrittsöffnung ist so klein, dass sie keine nennenswerten Schäden am Injektionsort hinterlassen, aber dennoch groß genug, um die erfindungsgemäße Zubereitung im Auge mit einer ausreichenden Geschwindigkeit zu applizieren. Wenn der Zylinder der Spritze im Durchmesser entsprechend angepasst ist, wird vermieden, dass sich im Inneren der Spritze bzw. der Kanüle ein Druck aufbaut, der bei der Injektion die Zubereitung unter zu starkem Druck in das Auge bringt. so dass die Zubereitung über den Applikationsort hinaus, z.B. hinter der Netzhaut, verteilt wird. Als besonders gut hinsichtlich der erwünschten Applikation haben sich Kanülen mit 20, 23, 25 oder 27 gauge, insbesondere solche mit 23 oder 25 gauge erwiesen. In einer bevorzugten Ausführungsform wird eine derartige Kanüle zusammen mit einer Spritze mit einem Zylinderdurchmesser von 3 bis 10 mm eingesetzt. Besonders bevorzugt sind Kanülen mit 23 oder 25 gauge, wenn die dynamische Viskosität der Zubereitung bei 25 °C und einer Scherrate von 10⁻¹ in einem Bereich von 1 bis 500 mPas liegt. Gerade dann ist das Zusammenspiel zwischen Kanüle und Zubereitung so gut, dass in ausreichend schneller Geschwindigkeit gleichmäßig eine genügend große Menge der erfindungsgemäßen Zubereitung am Applikationsort abgesetzt werden kann, ohne dass es durch Druckstauung zum explosionsartigen Austritt der Zubereitung aus der Kanüle kommt. Somit wird verhindert, dass die Zubereitung hinter den gewünschten Applikationsort injiziert wird, wodurch ein optimales Anfärben der Membran erzielt werden kann.

Die oben beschriebenen erfindungsgemäßen Zubereitungen und die zu ihrer Verabreichung bereitgestellten Spritzen ermöglichen es, im Auge gezielt Membranen - ILM und/oder ERM - anzufärben. Je nach eingesetztem Farbstoff ist es möglich entweder nur eine Art von Membran, d.h. nur ILM oder nur ERM anzufärben, oder aber beide Arten anzufärben. In einer Ausführungsform kann die erfindungsgemäße Zubereitung dazu verwendet werden, eine Negativfärbung der epiretinalen Membranen zu bewirken, um diese dann entfernen zu können. In dieser Ausführungsform wird eine Lösung eines Farbstoffs, z.B. Brillant Blau G, eingesetzt, der selektiv die ILM, nicht aber die ERM färbt. Auf diese Weise lasst sich die nicht gefärbte Membran (ERM) von der gefärbten Membran (ILM) unterscheiden und kann daher gut entfernt werden.

Die Erfindung wird noch durch die folgenden Beispiele, die Farbstofflösungen mit erhöhter Dichte und deren Herstellung beschreiben, erläutert, ohne sie darauf zu beschränken.

### Beispiel 1

0,025 g Brilliantblau G, 5 g Saccharose , 0,19 g Dinatriumhydrogenphosphat, 0,03 g Natriumdihydrogenphosphat und 0,82 g Natriumchlorid werden genau eingewogen und mit destilliertem Waser auf 100 g aufgefüllt. Die Rohstoffe werden in einer Glasflasche für 1 h bei maximal 60 °C behandelt, wobei eine homogene Lösung mit einer Farbstoffkonzentration von 0,25 g/L und einer Dichte von 1,023 g/cm³ entsteht.

### Beispiel 2

0,025 g Brilliantblau G, 5 g Trehalose , 0,19 g Dinatriumhydrogenphosphat, 0,03 g Natriumdihydrogenphosphat und 0,82 g Natriumchlorid werden genau eingewogen und mit destilliertem Waser auf 100 g aufgefüllt. Die Rohstoffe werden in einer Glasflasche für 1 h bei maximal 60 °C behandelt, wobei eine homogene Lösung mit einer Farbstoffkonzentration von 0,25 g/L und einer Dichte von 1,023 g/cm³ entsteht.

### Vergleichs-Beispiel 3

0,025 g Brilliantblau G, 0,19 g Dinatriumhydrogenphosphat, 0,03 g Natriumdihydrogenphosphat und 0,82 g Natriumchlorid werden genau eingewogen und mit einer Mischung aus destilliertem Wasser und D₂O auf 100 g aufgefüllt. Die Rohstoffe werden in einer Glasflasche für 1 h bei maximal 60 °C behandelt, wobei eine homogene Lösung mit einer Farbstoffkonzentration von 0,25 g/L, einer Dichte von 1,018 g/cm³ entsteht.

### Vergleichs-Beispiel 4

### Farbstoff+Glycerin

0,025 g Brilliantblau G, 0,19 g Dinatriumhydrogenphosphat, 0,03 g Natriumdihydrogenphosphat und 0,82 g Natriumchlorid werden genau eingewogen und mit einer Mischung aus destilliertem Wasser und 10 % Glycerin aufgefüllt. Die Rohstoffe werden in einer Glasflasche für 1 h bei maximal 60 °C behandelt, wobei eine homogene Lösung mit einer Farbstoffkonzentration von 0,25 g/L, einer Dichte von 1,027 g/cm³.

### Beispiel 5

Mit dem Verfahren, wie in den Beispielen 1 bis 4 beschrieben wurde eine Farbstofflösung mit folgender Zusammensetzung hergestellt

| Substanz | Einwaage soll ist in g | Einwaage ist in g |
|---|---|---|
| Polyvinylpyyrolidon | 6 | 6,0067 |
| Brilliant Blau G | 0,0125 | 0,0125 |
| Na₂HPO₄*2H₂O | 0,095 | 0,0950 |
| NaH₂PO₄*2H₂O | 0,015 | 0,0159 |
| NaCl | 0,41 | 0,4100 |
| Wasser | auf 50 g | auf 50 g |

Es wurde eine homogene Lösung mit einer Dichte von 1,028 g/cm³ und einer Viskosität von 7,38 mPas erhalten.

### Beispiel 6

Mit dem Verfahren, wie in den Beispielen 1 bis 4 beschrieben wurde eine Farbstofflösung mit folgender Zusammensetzung hergestellt

| Substanz | Einwaage soll ist in g | Einwaage ist in g |
|---|---|---|
| Methylcellulose E 10 M (2 wt.%) | 25 | 24,9986 |
| Brilliant Blau G | 0,0125 | 0,0125 |
| Na₂HPO₄*2H₂O | 0,095 | 0,0956 |
| NaH₂PO₄*2H₂O | 0,015 | 0,0151 |
| NaCl | 0,41 | 0,4099 |
| Wasser | auf 50 g | auf 50 g |

Es wurde eine homogene Lösung mit einer Dichte von 1,007 g/cm³ und einer Viskosität von 142,79 mPas erhalten.

Die in den Beispielen 1 bis 6 hergestellten Farbstofflösungen wurden zur Anfärbung der Membrana limitans interna im menschlichen oder tierischen Auge verwendet. Es wurde gefunden, dass sich alle sechs Lösungen sehr gut auftragen ließen und nach der Applikation sofort absanken und die ILM anfärbten Die Färbung war, bei gleicher Menge an Farbstoff, noch intensiver als mit einer zum Vergleich aufgetragenen Briliant Blau G Lösung, wie sie aus DE 10255601 bekannt ist.

## Patentansprüche

1. Wasserbasierte, biokompatible Zubereitung zur Verwendung bei der chirurgischen Behandlung am Auge, umfassend das selektive Anfärben der Membrana limitans interna (ILM) und/oder von epiretinalen Membranen (ERM) im menschlichen oder tierischen Auge und Entfernen der gefärbten Membran, enthaltend mindestens einen Farbstoff, ausgewählt aus der Gruppe bestehend aus: Triphenylmethanfarbstoffen, wobei die Zubereitung eine Dichte im Bereich von 1,01 g/cm³ bis 1,5 g/cm³ aufweist, wobei zum Einstellen der Dichte kein Monosaccharid oder reduzierendes Disaccharid verwendet wird, **dadurch gekennzeichnet, dass** die Zubereitung ein Mittel enthält, mit dem die Dichte eingestellt wird, wobei das Mittel ein nicht-reduzierendes Disaccharid, ein Polysaccharid, oder ein neutrales Polymer oder eine Kombination davon ist und dass die Osmolarität der hergestellten Lösungen im Bereich von 280-330 mosmol/L liegt.

2. Zubereitung zur Verwendung nach Anspruch 1, zusätzlich enthaltend einen Farbstoff ausgewählt aus der Gruppe bestehend aus Azofarbstoffen, Cyaninfarbstoffen und/oder Naturfarbstoffen oder deren Mischungen.

3. Zubereitung zur Verwendung nach Anspruch 1, wobei der Triphenylmethanfarbstoff Coomassie Violet R200 ist.

4. Zubereitung zur Verwendung nach einem der vorhergehenden Ansprüche zur Verwendung als Farbstoff für eine Negativdarstellung epiretinaler Membranen.

5. Zubereitung zur Verwendung nach einem der vorhergehenden Ansprüche, **dadurch gekennzeichnet, dass** die Zubereitung bei 25°C und einer Scherrate von 10 s⁻1 eine dynamische Viskosität im Bereich von 1 bis 500 mPas, bevorzugt in einem Bereich von 50 bis 275 mPas aufweist.

6. Zubereitung zur Verwendung nach einem der vorhergehenden Ansprüche, **dadurch gekennzeichnet, dass** die Osmolarität der hergestellten Lösungen im Bereich von 300 mosmol/L liegt.

7. Wasserbasierte, biokompatible Zubereitung zum in vivo selektiven Anfärben der Membrana limitans interna (ILM) und/oder von epiretinalen Membranen (ERM) im menschlichen oder tierischen Auge, enthaltend mindestens einen Triphenylmethanfarbstoff wobei die Zubereitung ein Mittel enthält, mit dem eine Dichte im Bereich von 1,01 g/cm³ bis 1,5 g/cm³ eingestellt wird, und wobei zum Einstellen der Dichte kein Monosaccharid oder reduzierendes Disaccharid verwendet wird, wobei das Mittel ein nicht-reduzierendes Disaccharid, ein Polysaccharid, oder ein neutrales Polymer oder eine Kombination davon ist **dadurch gekennzeichnet, dass** die Osmolarität der hergestellten Lösungen im Bereich von 280-330 mosmol/L liegt.

## Claims

1. A water-based, biocompatible preparation for use in surgical treatment on the eye, comprising the selective staining of the internal limiting membrane (ILM) and/or of epiretinal membranes (ERM) in the human or animal eye and removal of the stained membrane, containing at least one dye selected from the group consisting of: triphenylmethane dyes, wherein the preparation has a density in the range of 1.01 g/cm³ to 1.5 g/cm³, wherein for adjusting the density no monosaccharide or reduced disaccharide is used, **characterized in that** the preparation contains an agent that adjusts the density, wherein the agent is a non-reducing disaccharide, a polysaccharide or a neutral polymer or a combination thereof, and **characterized in that** the osmolarity of the solutions produced is in the range of 280-330 mosmol/l.

2. The preparation according to claim 1, additionally containing a dye selected from the group consisting of azo dyes, cyanine dyes and/or natural dyes or mixtures thereof.

3. The preparation according to claim 1, wherein the triphenylmethane dye is Coomassie Violet R200.

4. The preparation according to one of the preceding claims for use as a dye for a negative representation of epiretinal membranes.

5. The preparation according to one of the preceding claims, **characterised in that** the preparation has a dynamic viscosity in the range of 1 to 500 mPas, preferably in a range of 50 to 275 mPas, at 25°C and a shear rate of 10 s⁻¹.

6. The preparation according to one of the preceding claims, **characterised in that** the osmolarity of the solutions produced is in the range of 300 mosmol/l.

7. A water-based, biocompatible preparation for the in vivo selective staining of the internal limiting membrane (ILM) and/or of epiretinal membranes (ERM) in the human or animal eye, containing at least one triphenylmethane dye, wherein the preparation contains an agent that adjusts the density to a range of 1.01 g/cm³ to 1.5 g/cm³, and wherein for adjusting the density no monosaccharide or reduced disaccharide is used, wherein the agent is a non-reducing disaccharide, a polysaccharide or a neutral polymer or a combination thereof, and **characterized in that** the osmolarity of the solutions produced is in the range of 280-330 mosmol/l.

## Revendications

1. Préparation biocompatible aqueuse pour l'utilisation lors du traitement chirurgical sur l'œil, comprenant la coloration sélective de la membrane limitante interne (MLI) et/ou de membranes épirétiniennes (MER) dans l'œil humain ou animal et l'élimination de la membrane colorée, contenant au moins un colorant sélectionné parmi le groupe constitué de: colorants de triphénylméthane, dans laquelle la préparation présente une densité dans la plage de 1,01 g/cm³ à 1,5 g/cm³, dans laquelle aucun monosaccharide ou disaccharide réduit n'est utilisé pour le réglage de la densité, dans l'agent est un disaccharide non réducteur, un polysaccharide ou un polymère neutre ou une combinaison de ceux-ci, et **caractérisée en ce que** l'osmolarité des solutions fabriquées se situe dans la plage de 280 à 330 mosmol/L.

2. Préparation pour l'utilisation selon la revendication 1, contenant en outre un colorant sélectionné parmi le groupe constitué de colorants azoïques, colorants de cyanine et/ou colorants naturels ou leurs mélanges.

3. Préparation pour l'utilisation selon la revendication 1, dans laquelle le colorant de triphénylméthane est le Coomassie Violet R200.

4. Préparation pour l'utilisation selon une des revendications précédentes pour l'utilisation en tant que colorant pour une représentation négative de membranes épirétiniennes.

5. Préparation pour l'utilisation selon une des revendications précédentes, **caractérisée en ce que** la préparation présente à 25°C et à une vitesse de cisaillement de 10 s⁻¹ une viscosité dynamique dans la plage de 1 à 500 mPas, de préférence dans une plage de 50 à 275 mPas.

6. Préparation pour l'utilisation selon une des revendications précédentes, **caractérisée en ce que** l'osmolarité des solutions fabriquées se situe dans la plage à 300 mosmol/L.

7. Préparation biocompatible aqueuse pour la coloration sélective in vivo de la membrane limitante interne (MLI) et/ou de membranes épirétiniennes (MER) dans l'œil humain ou animal, contenant au moins un colorant de triphénylméthane, dans laquelle la préparation contient un agent avec lequel une densité dans la plage de 1,01 g/cm³ à 1,5 g/cm³ est réglée, et dans laquelle aucun monosaccharide ou disaccharide réduit n'est utilisé pour le réglage de la densité dans l'agent est un disaccharide non réducteur, un polysaccharide ou un polymère neutre ou une combinaison de ceux-ci, e **caractérisée en ce que** l'osmolarité des solutions fabriquées se situe dans la plage de 280 à 330 mosmol/L.
